# EUROPEAN PATENT APPLICATION

(11) **EP 4 227 406 A1**
(43) Date of publication of application: **16.08.2023**
(21) Application number: 21877123.6
(22) Date of filing: 08.10.2021
(51) Int. Cl.: C12N 5/10, C12N 15/63, C12N 15/86, C07K 14/47

(54) **METHOD FOR STIMULATION OF MESENCHYMAL CELLS TO INDUCE IMMUNOMODULATORY FACTOR EXPRESSION**

(30) Priority: 08.10.2020 CO 20012570
(71) Applicant: INSTITUTO DISTRITAL DE CIENCIA BIOTECNOLOGIA E INNOVACIÓN EN SALUD - IDCBIS, Bogotá (CO)
(72) Inventor: CAÑAS ARBOLEDA, Mariana, 11-501 Bogota (CO); CRUZ BARRERA, Monica Liliana, 91- 36 Bogotá (CO); SALGUERO LÓPEZ, Gustavo Andres, 28 Bogotá (CO)
(74) Representative: Clarke, Modet y Cía., S.L.
(86) International application number: PCT/IB2021/059254
(87) International publication number: WO 2022/074624

(57) **Abstract**

The present invention relates to a method of stimulating mesenchymal stromal cells to induce the expression of immunomodulatory factors, and a pharmaceutical composition comprising a population of mesenchymal stromal cells obtained by said method. The invention also relates to applications of said pharmaceutical composition in conditions of an autoimmune nature or associated with acute or chronic inflammation.

## Description

### TECHNICAL FIELD

The present invention relates to the general field of medicine, particularly to cell therapy and regenerative medicine, and more particularly to a method of stimulating mesenchymal stromal cells to induce the expression of immunomodulatory factors, and a pharmaceutical composition comprising a population of mesenchymal stromal cells obtained by said method. The invention also relates to applications of said pharmaceutical composition in conditions of an autoimmune nature or associated with acute or chronic inflammation.

### BACKGROUND OF THE INVENTION

Cell therapy strategies based on the use of mesenchymal stromal cells (MSCs) have become an expanding tool for regenerative medicine. The growing clinical evidence accumulated in recent years has demonstrated the feasibility of the application of MSC-based therapies in terms of biosafety and therapeutic potential in a variety of pathologies associated with autoimmunity, chronic inflammation and osteoarticular regeneration. Multiple clinical trials have been developed with MSC-based therapies for a variety of chronic and acute inflammatory conditions including type I diabetes mellitus, rheumatoid arthritis, multiple sclerosis or graft-versus-host disease. In line with the above, MSCs have shown a robust biosafety profile and objective clinical responses that would indicate adequate efficacy, evidencing in many cases control of immune and inflammatory responses, symptoms relief and improved quality of life of treated patients [Kieran, K., et al., A Systematic Review of Clinical Studies Investigating Mesenchymal Stem Cells for Fracture Non-Union and Bone Defects. Current Stem Cell Research & Therapy, 2018. 13(4): p. 284- 291]. There is recent evidence that the molecular machinery of MSCs induces multi-faceted immune responses at the cellular and tissue level, making them an attractive platform for developing cell-based immune therapies.

One of the main aspects that impacts not only the therapeutic efficacy but also the manufacturing process of cell therapies with human MSCs is the introduction of alternative sources for obtaining them, in order to improve the availability and feasibility of scaling up the product for clinical use. MSCs applied to immune therapy have been successfully isolated and expanded from a variety of sources, such as bone marrow or adipose tissue. Recently, however, an attractive source of MSCs has been studied, which is the umbilical cord (UC), a commonly discarded by-product after childbirth. Human UC-derived MSCs (UC-MSCs) have gained special attention due to their high availability and easy accessibility. It should be noted that, given their fetal origin, UC-MSC have particular advantages, such as enhanced multipotency, increased differentiation and greater proliferative capacity [Jin, H.J., et al., Comparative analysis of human mesenchymal stem cells from bone marrow, adipose tissue, and umbilical cord blood as sources of cell therapy. International Journal of Molecular Sciences, 2013. 14(9): p. 17986-18001], [Wu, M., et al., Comparison of the Biological Characteristics of Mesenchymal Stem Cells Derived from the Human Placenta and Umbilical Cord. Scientific Reports, 2018. 8(1): p. 5014], [Hsieh, J.-Y., et al., Functional Module Analysis Reveals Differential Osteogenic and Stemness Potentials in Human Mesenchymal Stem Cells from Bone Marrow and Wharton's Jelly of Umbilical Cord. Stem Cells and Development, 2010. 19(12): p. 1895-1910].]. In addition, recent evidence has also suggested more potent immunomodulatory effects of UC-MSCs *in vitro* and *in vivo* [Kim, J.-H., et al., Comparison of Immunological Characteristics of Mesenchymal Stem Cells from the Periodontal Ligament, Umbilical Cord, and Adipose Tissue. Stem cells international, 2018. 2018: p. 8429042-8429042].

The introduction of UC-MSCs as a therapeutic tool has opened new spaces for clinical use in the allogeneic setting, taking into account that the use of MSCs for clinical applications has been mainly restricted to the autologous settings [Oliver-Vila, I., et al., Evaluation of a cell-banking strategy for the production of clinical grade mesenchymal stromal cells from Wharton's jelly. Cytotherapy, 2016. 18(1): p. 25-35]. Therefore, the establishment of clinical-grade UC-MSC banks has become feasible given the advantage of the immediate availability of umbilical cord tissues for the production of MSC-based therapies, particularly in already existing public cord blood registry facilities. As long as the allogeneic use of MSCs proves to be effective and safe, clinical-grade UC-MSC banks can provide unlimited access to immune cell therapies.

The state of the art shows processes for the expansion of MSCs derived from various sources and their possible applications. There are also studies in which UC-MSCs are used as a potential therapy tool in rheumatologic and other diseases. In some cases they are cells genetically engineered to constitutively produce a growth factor. Depending on the clinical application, other preparation solutions in biogels, or the combination of multiple "*cells with regenerative potential*" are also evident.

For example, Patent US20180236003A1 discloses a composition comprising a medium enriched with a high-molecular weight glycoconjugate with or without expanded adipose tissue-derived MSCs, as well as the method for preparing such medium. Additionally, it relates to a method for preparing a composition comprising a stable secretome, wherein said stability comprises retaining at least 60% activity after one month at room temperature. Such a stable secretome comprises one or more factors selected from among the following IFN-γ, IL-8, IL-9, IL-12, IL-15, TNF-α, IL-10, MCP-1, RANTES, GM-CSF, IP-10, PDGF-bb, VEGF, IL-6, and VEGF. Similarly, a method for large-scale production of cultured MSCs and a method for treating an inflammatory condition are disclosed, or for relieving pain associated with an inflammatory condition, or for treating neuropathic pain, comprising topically administering a therapeutically effective amount of a composition comprising media conditioned with *in vitro* MSC culture.

Patent CN108588017 discloses a method for separating and culturing umbilical cord-derived mesenchymal stromal cells and the application of MSCs obtained by the aforementioned process, together with PRP and hyaluronic acid in bones and joints for the treatment of osteoarthritis.

Patent US20180325957A1 discloses a composition comprising a suspension of immunosuppressive mesenchymal stromal cells derived from human adipose tissue at a minimum concentration of 1.5×10⁷ cells/mL, further characterized in that after stimulation with interferon-γ for 3 days, they express 80% more CD274 and 25-fold more CD54. Likewise, D3 discloses the method for obtaining said composition.

Therefore, as research into the immunomodulatory functions of UC-MSCs continues to expand, interest in better and more efficient strategies for immune cell therapy persists, as well as the need to develop processes for cell production and scale-up for clinical use.

### BRIEF DESCRIPTION OF THE INVENTION

The present invention relates to a method of stimulating mesenchymal stromal cells to induce the expression of immunomodulatory factors, and a pharmaceutical composition comprising a population of mesenchymal stromal cells obtained by said method. The invention also relates to applications of said pharmaceutical composition in conditions of an autoimmune nature or associated with acute or chronic inflammation.

The method of the present invention comprises culturing and expanding MSCs in a culture medium supplemented with immunomodulation-stimulating and immunomodulatory phenotype-inducing factors, as well as the addition of inflammatory cytokines, or exposure to immunoactivated leukocytes from UC-MSCs to enhance their immune system modulating effect.

### BRIEF DESCRIPTION OF FIGURES

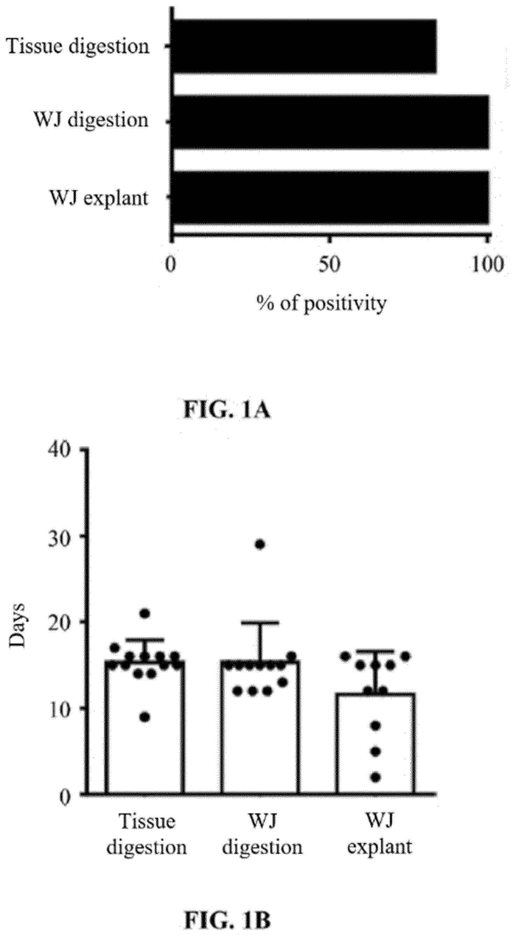

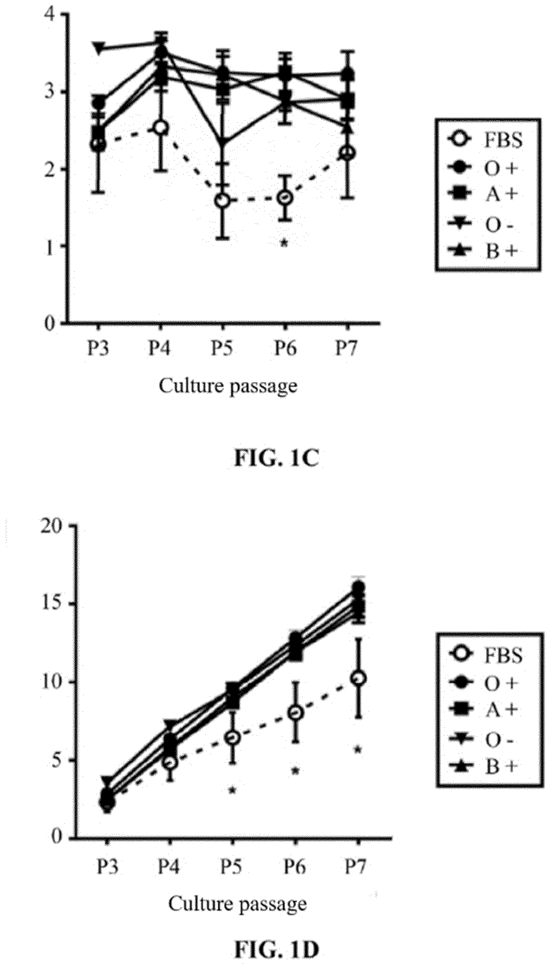

**FIG. 1** **Methods of isolation and culture of UC-MSCs.** (A) Three UC-MSCs isolation methodologies (enzymatic digestion of total cord tissue, Wharton's jelly (WJ) digestion and WJ explant) were tested to determine the efficiency and yield of cell isolation from umbilical cord tissues. (B) Time (in days) to obtain the first explant in the three methodologies. (C) Population doubling level per passage of UC-MSCs supplemented with hLP of various blood groups compared to fetal bovine serum (FBS). (D) Cumulative population doublings of various UC-MSC donors compared to FBS.

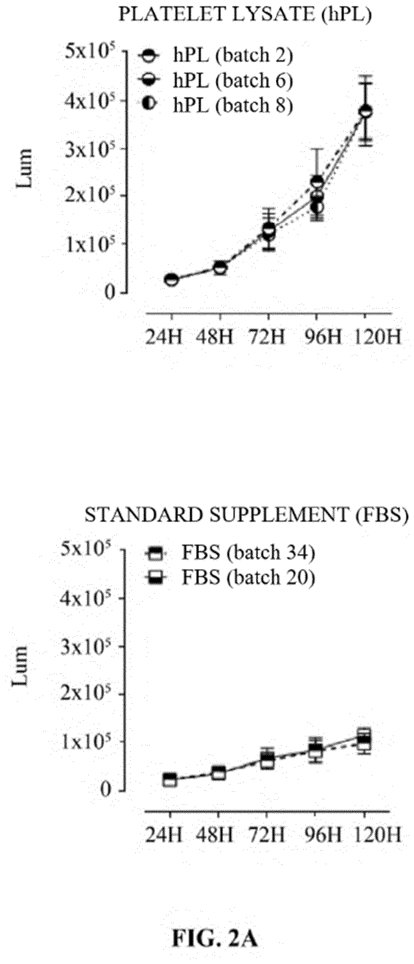

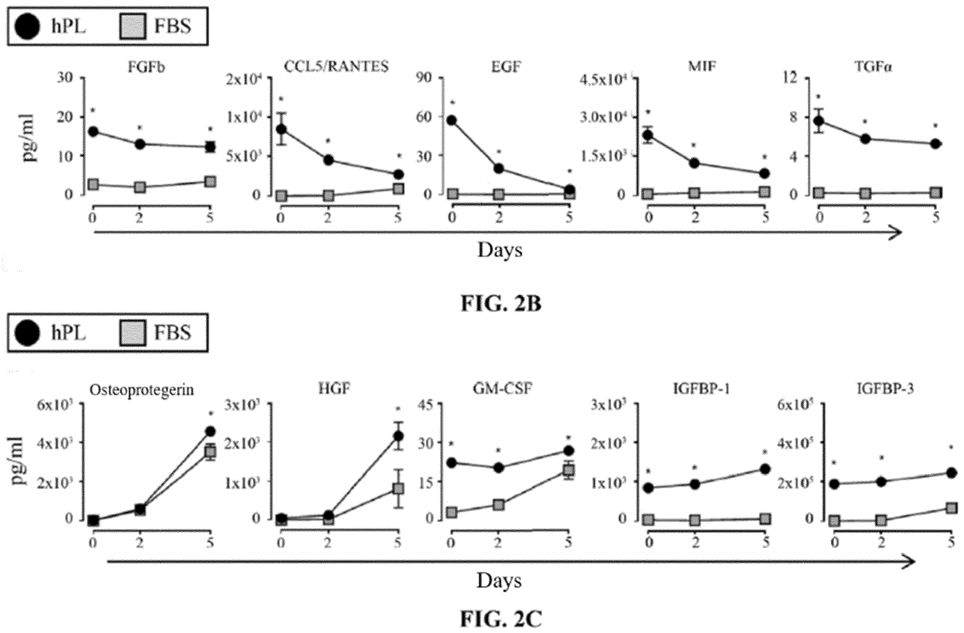

**FIG. 2** **Tests based on the stimulation of growth and secretion of UC-MSC-immunomodulatory factors with culture medium enriched with an in-house preparation of hLP-based supplement, compared to standard Fetal Bovine Serum (FBS)-based supplement.** (A) shows the effect of the supplement in relation to cell growth-expansion, demonstrating superiority of hLP over human serum and fetal bovine serum. (B) shows the consumption of stimulatory factors by UC-MSCs indicating active metabolism and specific stimulation of UC-MSCs during expansion. (C) demonstrates the sustained release of immunomodulatory and regenerative signals by UC-MSCs by applying the developed expansion methodology. *=hPL vs. FBS, p<0.05.

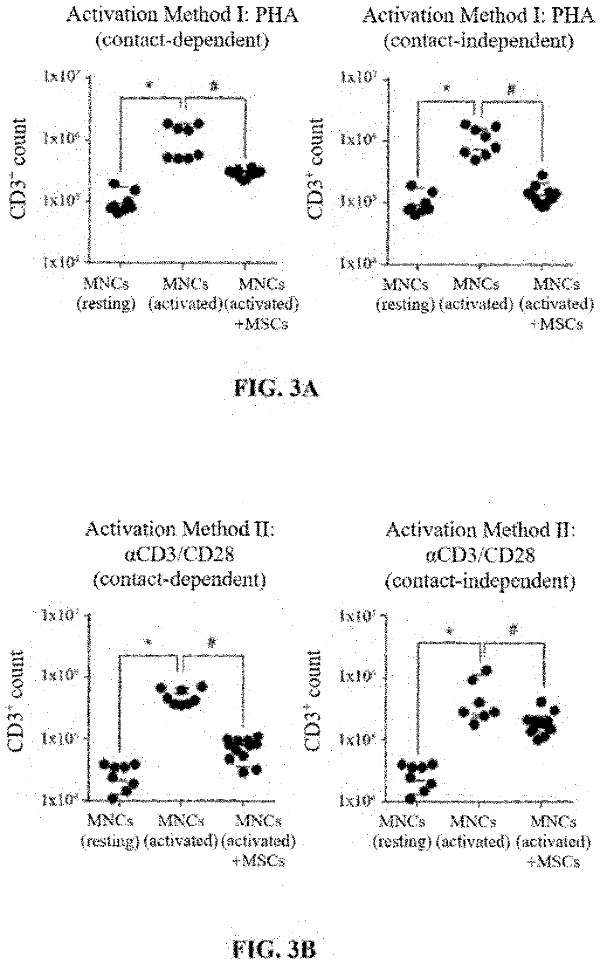

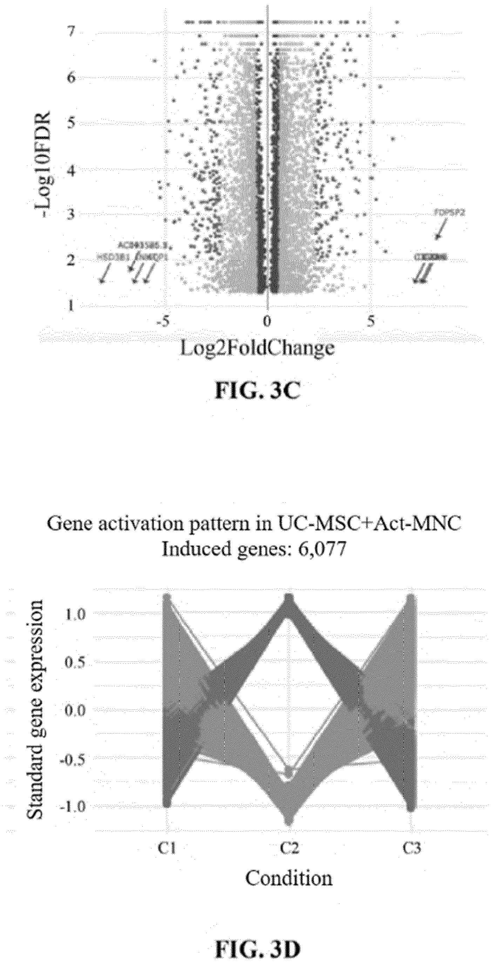

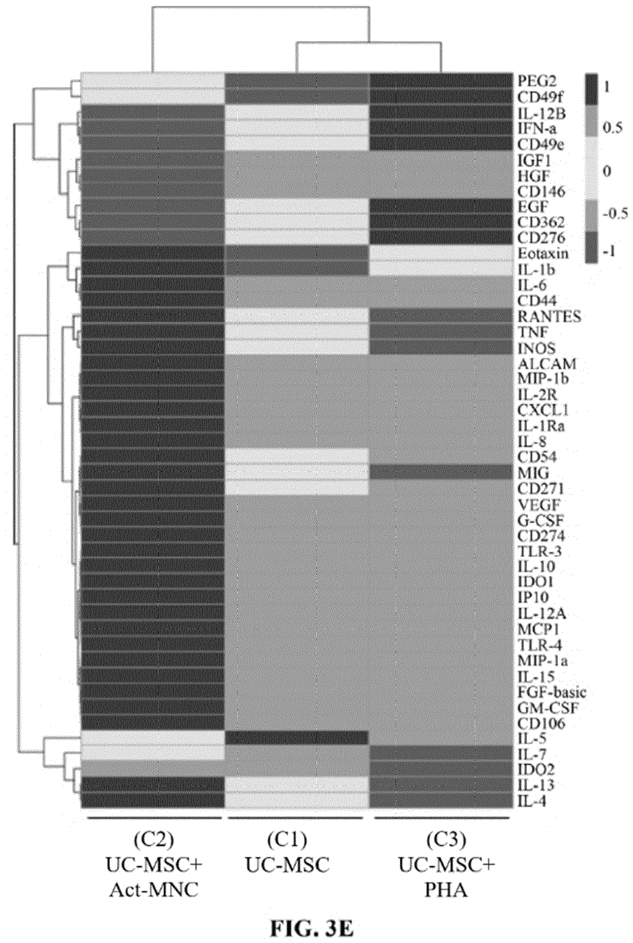

**FIG. 3** **Results of *in vitro* tests on the anti-inflammatory effect of UC-MSCs.** Immunoassays based on the suppression of T-lymphocyte proliferation (CD3+) evaluated at day five (5) post-culture based on the absolute determination of the number of CD3+ contained in mononuclear cells (MNCs) that are non-activated (resting), activated, or activated and co-cultured with UC-MSCs (MNC+MSC). (A) activated with phytohemagglutinin (PHA). (B) activated with anti-CD3/CD28 (αCD3/CD28). = MNCs (resting) vs. MNCs (activated), p<0.05. #=MNCs (activated) vs. MNCs (activated)+MSCs, p<0.05. In both immunostimulation models, absolute inhibition of the proliferation of activated T lymphocytes is evident, indicating a potent immunosuppressive effect of MSC-MSCs on immune component cells in a state of inflammation/immunoactivation.
   **Induction of changes at the transcriptomic level in UC-MSCs resting or under immunomodulation** Induction of changes at the transcriptomic level in UC-MSCs resting or under immunomodulation (C) Volcano plot of differentially expressed genes (DEG) among the three experimental conditions (C1: cells in basal state or UC-MSCs; C2: MNCs (activated)+UC-MSCs; C3: UC-MSCs not immunoactivated but exposed to the leukocyte immunostimulation drug PHA+UC-MSCs. Induction of 6,077 genes was observed in the immunoactivated cells. (D) Expression patterns of differentially expressed genes. Approximately 2,600 were induced up-regulated and 3,500 down-regulated in UC-MSCs in an immunomodulated state. (E) Standardized expression levels of immunomodulatory genes in UC-MSCs in immunomodulation (C3) vs. Controls (C1 and C2). Among the predominantly induced genes were found to be genes actively involved in immune signaling pathways characterized by suppression/immunomodulation of immune responses.

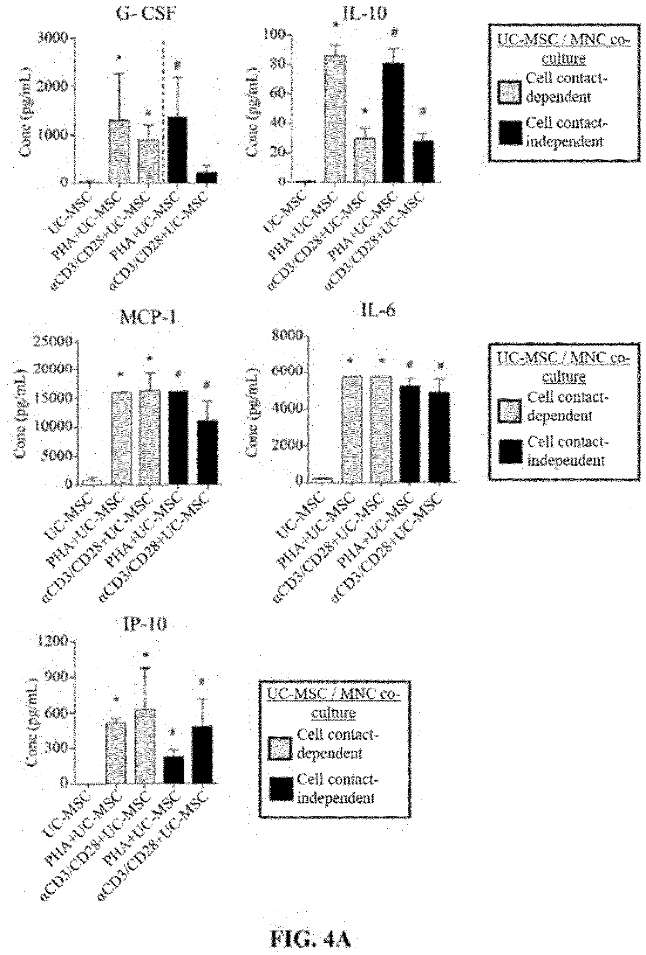

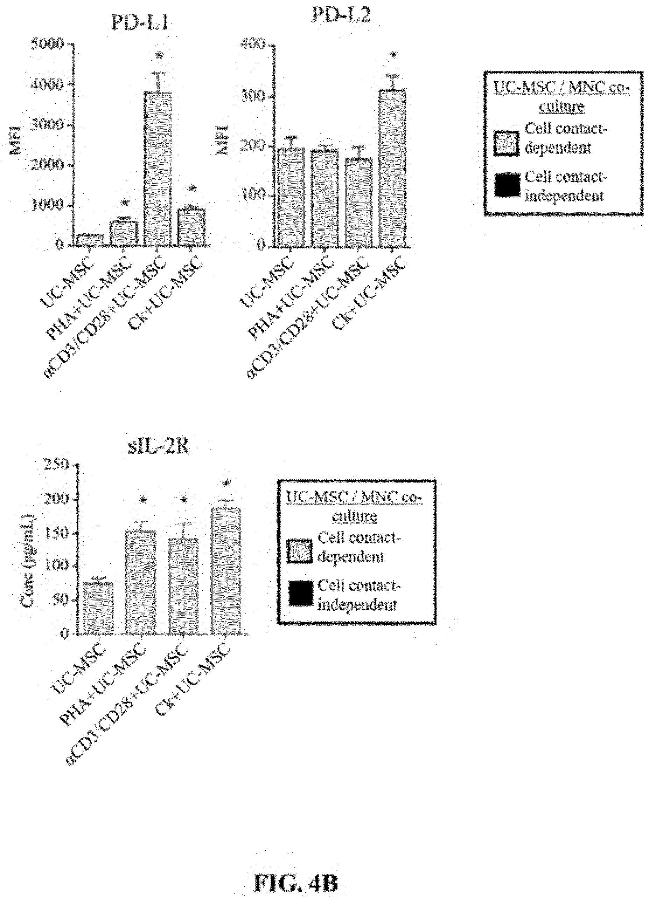

**FIG. 4** **Production of immunomodulatory signals by UC-MSCs in inflammation models** (A) UC-MSCs were exposed to mononuclear cells (MNCs) immuno-activated with phytohemagglutinin (PHA) or anti-CD3/CD28 in a cell contact configuration (gray bars), or separated across a 0.4 µm membrane (black bars) and the production of G-CSF, IL-10, MCP-1, IL-6 and IP-10 factors was assessed on the fifth day of stimulation. *=UC-MSCs vs. PHA+UC-MSCs,αCD3/CD28+UC-MSCs or CK+UC-MSCs in a cell contact-dependent configuration, p<0.05. #=UC-MSCs vs. PHA+UC-MSCs or αCD3/CD28+UC-MSCs in a cell contact-independent configuration, p<0.05. (B) Additionally, it could be determined that the UC-MSC preparation exposed to a pro-inflammatory environment induced the significant increase of potent immunomodulatory signals such as soluble IL-2 receptor (IL-2R) production and expression of immune checkpoint stimulatory molecules PD-L1 and PD-L2 in UC-MSCs stimulated with IL-1b and TNFα (CK).

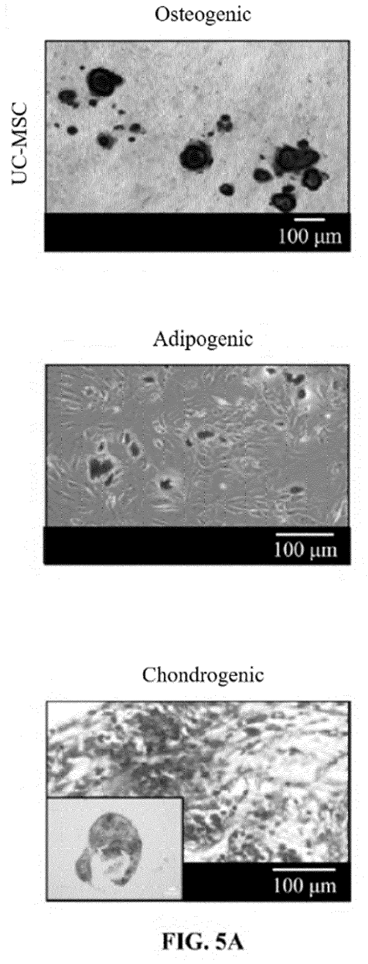

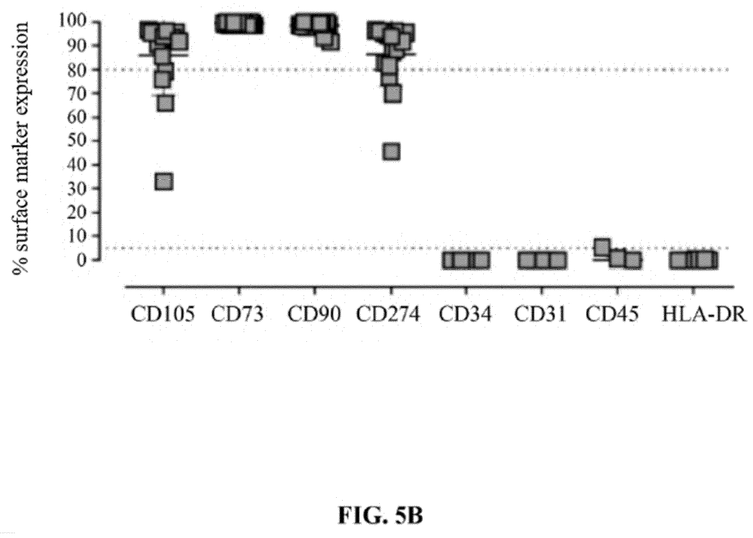

**FIG. 5** **Characterization of the UC-MSC population.** (A) Representative micrographs of UC-MSCs from different donors (n=4) that were characterized in terms of differentiation potential towards osteogenic, chondrogenic and adipogenic lineage. (B) Characterization of the immunophenotype of UC-MSCs based on the evaluation of mesenchymal identity markers (CD90, CD73, CD105, CD274) and exclusion of hematopoietic/endothelial markers (CD45, CD34, HLA-DR and CD31).

### DETAILED DESCRIPTION OF THE INVENTION

For the interpretation of this description, the following definitions shall apply, and where appropriate, terms used in the singular form shall also include the plural form. Thus, the terms used in the description have the following meanings unless the context clearly indicates otherwise:
**Peripheral blood mononuclear cells (PBMC):** cells contained in mammalian hematopoietic compartments such as bone marrow, peripheral blood, and spleen, and comprising mostly the mononuclear cell component of the immune and hematopoietic system, where lymphocytes, monocytes, dendritic cells and Natural Killer cells are found. These cells can be isolated through methodologies based on the use of colloids such as Ficoll and centrifugation gradients that separate the mononuclear component from the polymorphonuclear component and the erythroid component of these fluids.

**Autologous use:** In the context of organ, tissue, and cell transplantation, it refers to the origin of the graft (cells, tissue, organ), which in this case is from the same individual. In the opposite case, when the tissue comes from another individual, it is called "allogeneic use".

**Heterologous use:** In the context of tissue and cell transplantation, it refers to the final use of the graft to replace, substitute or improve the function of the recipient tissue. In this case, heterologous use indicates that the (cell or tissue) graft will not be used in the recipient for the original function or structure. This is the case of the use of amniotic membrane for the repair of corneal or epidermal lesions, the use of hematopoietic progenitor cells for the treatment of cerebral or cardiac hypoxia, etc.

**Human platelet lysate (hPL):** cell culture supplement derived from hemocomponents, specifically platelet-rich plasma or platelet concentrates obtained from blood donors, usually from a blood bank.

**Immunomodulatory factors:** factors produced and secreted by various cells of the immune component and connective tissue, the main effect of which is the regulation of the inflammatory activity of immune cells during the inflammatory phase of an injury or infection process in the affected tissue.

**Wharton's jelly (WJ):** corresponds to most of the tissue that makes up the umbilical cord in mammals. This tissue composed of collagen and proteoglycans surrounds the blood vessels of the cord. It is also the main source of cord mesenchymal stromal cells.

**DMEM (Dulbecco's Modified Eagle's medium):** It is a culture medium consisting of a standard formulation of amino acids, sugar, vitamins, and ions necessary for the growth of various cell types. It is the standard medium used for the growth of mesenchymal stromal cells of any origin.

### Mesenchymal stromal cells (MSCs)

Mesenchymal stromal cells (MSCs) consist of a heterogeneous population of multipotent cells located in the interstitium of all human tissues, which proliferate *in vitro* adhered to plastic, have a fibroblast-like morphology, and can differentiate into cells of the mesodermal lineage such as osteocytes, chondrocytes, and adipocytes; they have been considered an important tool for cell therapy of various pathological conditions with an inflammatory component and tissue damage.

Mesenchymal stromal cells (MSCs) are critical for modulating immune responses in the context of inflammation. Based on evidence accumulated over the last decade, MSCs have become an attractive platform for developing cell-based immune therapies by harnessing their molecular machinery to drive multi-faceted immune responses at the cellular and tissue level [Pers, Y.M., et al., Mesenchymal stem cells for the management of inflammation in osteoarthritis: state of the art and perspectives. Osteoarthritis and Cartilage, 2015. 23(11): p. 2027-2035]. MSC-based therapies have been used clinically in several trials, enrolling more than 32,000 patients with a variety of acute and chronic inflammatory conditions, including type I diabetes mellitus, rheumatoid arthritis, multiple sclerosis, or graft-versus-host disease (ClinicalTrials.gov).

Current understanding of the molecular mechanisms of MSC-modulated immunosuppression points to local injury or inflammation as triggers to induce proliferation/activation of regulatory T cells, anergy of effector T cells, modulation of macrophages and dendritic cells through activation of molecular pathways involving release of soluble factors or metabolic alteration of immune cellular components [Wang, M., Yuan, Q. & Xie, L. Mesenchymal stem cell-based immunomodulation: Properties and clinical application. Stem Cells Int. 2018, (2018)]. Although the mechanisms by which MSCs exert immune suppression are not fully understood, *in vitro* and *in vivo* data indicate that MSCs act on different cell subsets involved in the initiation and maintenance of immune responses at the local and systemic levels [Bouffi, C., Bony, C., Courties, G., Jorgensen, C. & Noël, D. IL-6-dependent PGE2 secretion by mesenchymal stem cells inhibits local inflammation in experimental arthritis. PLoS One 5, (2010)]. MSC can restrict T and B lymphocyte proliferation and suppress their effector activity [Laing, A. G. et al. Mesenchymal stem cells inhibit T-cell function through conserved induction of cellular stress. PLoS One 14, 1-13 (2019)]. Similarly, the differentiation, antigen presentation and costimulatory function of dendritic cells, as well as the inflammatory activity of macrophages are altered in the presence of MSCs [Jiang, X. X. et al. Human mesenchymal stem cells inhibit differentiation and function of monocyte-derived dendritic cells. Blood 105, 4120-4126 (2005)].

While there is debate as to whether or not MSC-linked immune suppression mechanisms depend on cell contact, there is a broad consensus that secreted factors play a key role during MSC-mediated immune suppression. However, the great complexity of the immune modulation networks governing the cellular and molecular matrix by MSCs remains to be solved in order to develop new molecular tools and applications in the field of MSC-based therapies.

Despite their homogeneity in terms of identity and potency, MSCs of different origins share biological characteristics, but differ in other aspects such as the immunophenotype. For example, MSCs obtained from adipose tissue are positive in early passages for CD34, while MSCs from other sources do not express this marker [Cassatella, M.A., et al., Toll-like receptor-3-activated human mesenchymal stromal cells significantly prolong the survival and function of neutrophils. Stem Cells, 2011. 29(6): p. 1001-11.]. Kern et al. compared MSCs isolated from bone marrow, adipose tissue and umbilical cord tissue and found that bone marrow-derived MSCs have the lowest proliferation capacity, in terms of proliferation rate and number of population doublings, while umbilical cord MSCs show the highest proliferation rate. Similarly, the differentiation potential of MSCs also differed with respect to cell origin, with MSCs isolated from dental pulp differentiating preferentially into dentin rather than bone in *in vivo* assays [Gronthos, S., et al., Stem cell properties of human dental pulp stem cells. J Dent Res, 2002. 81(8): p. 531-5]. Bone marrow MSCs show a marked tendency to differentiate into chondrocytes and osteocytes compared to adipose tissue MSCs, which are more efficient in stimulating angiogenesis. Finally, with respect to gene expression analyses, MSCs show a high expression of genes associated with osteogenic differentiation for bone marrow MSCs, while umbilical cord MSCs show high expression of genes involved in angiogenesis [Panepucci, R.A., et al., Comparison of gene expression of umbilical cord vein and bone marrow-derived mesenchymal stem cells. Stem Cells, 2004. 22(7): p. 1263-78]. *In vitro* immunomodulation assays have shown that although bone marrow and adipose tissue MSCs have similar immunomodulatory effects by suppressing peripheral blood mononuclear cell proliferation and inhibiting monocyte differentiation into dendritic cells, adipose tissue MSCs have a more potent immunomodulatory effect in a dose-response relationship compared to bone marrow MSCs. This may be explained by their high metabolic activity, resulting in a high production of cytokines involved in immunosuppressive and anti-inflammatory mechanisms. Adipose tissue MSCs are genetically and morphologically more stable in long-term culture, show low senescence rate and high proliferative capacity, in addition to maintaining the differentiation potential compared to bone marrow MSCs [Hua, J., et al., Small Molecule-Based Strategy Promotes Nucleus Pulposus Specific Differentiation of Adipose-Derived Mesenchymal Stem Cells. Mol Cells, 2019. 42(9): p. 661-671.].

A major difficulty for the clinical translation of MSC-based products is the need to achieve adequate doses (number of cells) to obtain clinical effects. In this sense, the easy isolation and expansion potential are decisive for the selection of the best source of these cells. The MSCs that have been most widely used and studied are those obtained from bone marrow and adipose tissue. The amount that can be obtained from bone marrow is limited, ranging from 0.001 to 0.01%. [Pittenger MF, Mackay AM, Beck SC, Jaiswal RK, Douglas R, Mosca JD, Moorman MA, Simonetti DW, Craig S, Marshak DR. Multilineage potential of adult human mesenchymal stem cells. Science. 1999; 284(5411):143-7], while 5×10³ cells can be isolated in 1 g of adipose tissue [Fraser JK, Wulur I, Alfonso Z, Hedrick MH. Fat tissue: an underappreciated source of stem cells for biotechnology. Trends Biotechnol. 2006; 24(4):150-4. Epub 2006 Feb 20]. However, the efficiency of MSCs obtained from WJ is in the range of 1 to 5×10⁴ cells per centimeter of umbilical cord [Weiss ML, Medicetty S, Bledsoe AR, Rachakatla RS, Choi M, Merchav S, Luo Y, Rao MS, Velagaleti G, Troyer D. Human umbilical cord matrix stem cells: preliminary characterization and effect of transplantation in a rodent model of Parkinson's disease. Stem Cells. 2006; 24(3):781-92. Epub 2005 Oct 13]. In addition, adult MSCs, as a consequence of the time present in the body, are susceptible to the accumulation of cellular damage, which can lead to cell death, senescence or loss of regenerative function, and in extreme cases to neoplastic transformation. On the other hand, neonatal tissue MSCs such as WJ are safe from these factors [Kalaszczynska I, Ferdyn K. Wharton's jelly derived mesenchymal stem cells: future of regenerative medicine? Recent findings and clinical significance. Biomed Res Int. 2015; 2015:430847. doi: 10.1155/2015/430847. Epub 2015 Mar 15]. It has been recorded that the age of the MSC donor affects several properties of the cells [Stenderup K, Justesen J, Clausen C, Kassem M. Aging is associated with decreased maximal life span and accelerated senescence of bone marrow stromal cells. Bone. 2003 Dec;33(6):919-26], for example, Duscher et al. demonstrated that the age of the adipose tissue MSC donor compromises the ability of the cells to support the formation of vascular networks [Duscher D, Rennert RC, Januszyk M, Anghel E, Maan ZN, Whittam AJ, Perez MG, Kosaraju R, Hu MS, Walmsley GG, Atashroo D, Khong S, ButteAJ, GurtnerGC. Aging disrupts cell subpopulation dynamics and diminishes the function of mesenchymal stem cells. Sci Rep. 2014 Nov 21;4:7144. doi: 10.1038/srep07144.]. In the case of bone marrow MSCs, their potential to enhance the engraftment of other cells is implicated. Ko ci et al. demonstrated that the donor lifestyle can also affect cell potential; thus, bone marrow MSCs obtained from obese mice showed reduced adipogenic, osteogenic and chondrogenic differentiation potential and adipose tissue MSCs showed increased adipogenic and osteogenic differentiation and reduced chondrogenic potential. In view of the above, it can be deduced that the microenvironment of various diseases can influence MSCs, therefore, the isolation of MSCs from patients with metabolic diseases may not be useful as an autologous resource for cell therapy [Ko ci Z, Turnovcová K, Dubský M, Baranovi cov6 L, Holá n V, Chudi ckov6 M, Sykov6 E, Kubinová S. Characterization of human adipose tissue-derived stromal cells isolated from diabetic patient's distal limbs with critical ischemia. Cell Biochem Funct. 2014 Oct;32(7):597-604. doi: 10.1002/cbf.3056. Epub 2014 Sep 23].

Much of the current knowledge about the biological and therapeutic properties of MSCs has been generated from studies performed in bone marrow. However, the use of other alternative tissue sources such as adipose tissue, placenta, skin, dental pulp, umbilical cord blood, amniotic fluid, umbilical cord perivascular cells, WJ, synovial tissue, breast milk and menstrual blood, has begun to gain interest given the ease of collection of some of these tissues, avoiding the use of invasive techniques, such as the bone marrow aspiration procedure, during their isolation.

### Umbilical cord MSCs (UC-MSCs)

UC-MSCs are promising candidates for cell therapy due to their potent multilineage differentiation, enhanced self-renewal capacity, and immediate availability for clinical use. Clinical experience has demonstrated satisfactory biosafety profiles and the feasibility of the UC-MSCs application.

A robust *in vitro* immune assay was developed to measure various facets of immune responses triggered by UC-MSCs, integrating whole transcriptome, secretome and cellular responses under experimental immune modulation. Extensive analyses were conducted to validate already known molecular pathways involved in MSC-mediated immune modulation and to identify new candidates for the control of inflammation and immune activation by UC-MSCs.

Subramanian et al. characterized MSCs isolated from different compartments of the umbilical cord: amnion, subamnion, perivascular, WJ, and the whole cord fragment. It has been reported that WJ MSCs are superior to those isolated from the other compartments in terms of clinical utility because their isolation is simple, rapid and easy to standardize, they exhibit reduced contamination from other cells, are rich in progenicity characteristics, can be generated in large quantities with minimal manipulation, are highly proliferative, and have broad and efficient differentiation potential [Subramanian A, Fong C-Y, Biswas A, Bongso A. Comparative Characterization of Cells from the Various Compartments of the Human Umbilical Cord Shows that the Wharton's Jelly Compartment Provides the Best Source of Clinically Utilizable Mesenchymal Stem Cells. Pera M, ed. PLoS ONE. 2015;10(6):e0127992].

### Method for obtaining a population of immunomodulatory MSCs

### Isolation of UC-MSCs

For the purposes of the present invention, the MSCs can be isolated from bone marrow aspirates, adipose tissue, dental pulp, umbilical cord blood, placental tissue, menstrual fluid or amniotic fluid. In a particular modality, MSCs are isolated from umbilical cord (UC-MSCs), more particularly from WJ.

In general, a number of methods for the isolation of MSCs from virtually any tissue have been described in detail. Thus, since the last decade, MSCs have been isolated from bone marrow [Herzog, E. L., Chai, L. and Krause, D. S. (2003). Plasticity of marrow-derived stem cells. Blood 102, 3483-3493.], adipose tissue [Zuk, P. A., Zhu, M., Mizuno, H., Huang, J., Futrell, J. W., Katz, A. J., Benhaim, P., Lorenz, H. P. and Hedrick, M. H. (2001). Multilineage cells from human adipose tissue: implications for cell-based therapies. Tissue Eng. 7, 211-228], tendinous tissue[Salingcarnboriboon, R., Yoshitake, H., Tsuji, K., Obinata, M., Amagasa, T., Nifuji, A. and Noda, M. (2003). Establishment of tendon-derived cell lines exhibiting pluripotent mesenchymal stem cell-like property. Exp. Cell Res. 287, 289-300], periodontal ligament [Seo, B. M., Miura, M., Gronthos, S., Bartold, P. M., Batouli, S., Brahim, J., Young, M., Robey, P. G., Wang, C. Y. and Shi, S. (2004). Investigation of multipotent postnatal stem cells from human periodontal ligament. Lancet 364, 149-155], synovial membrane [De Bari, C., Dell'Accio, F., Tylzanowski, P. and Luyten, F. P. (2001). Multipotent mesenchymal stem cells from adult human synovial membrane. Arthritis Rheum. 44, 1928-1942], lung [Sabatini, F., Petecchia, L., Tavian, M., de Villeroche, V. J., Rossi, G. A. and Brouty-Boye, D. (2005). Human bronchial fibroblasts exhibit a mesenchymal stem cell phenotype and multilineage differentiating potentialities. Lab. Invest. 85, 962-971], perivascular tissue [Farrington-Rock, C., Crofts, N. J., Doherty, M. J., Ashton, B. A., Griffin-Jones, C. and Canfield, A. E. (2004). Chondrogenic and adipogenic potential of microvascular pericytes. Circulation 110, 2226-2232] among others [da Silva Meirelles, L., P.C. Chagastelles, and N.B. Nardi, Mesenchymal stem cells reside in virtually all post-natal organs and tissues. J Cell Sci, 2006. 119(Pt 11): p. 2204-13]. Obtaining MSCs from UC has several advantages as it is a tissue removed by non-invasive methods and considered a medical waste.

### MSC culture and expansion

### Culture medium

According to the present disclosure, the method of obtaining a population of UC-MSCs possessing potent immunomodulatory properties comprises culturing and expanding UC-MSCs in a culture medium supplemented with immunomodulatory stimulatory factors, adding cytokines or exposure to immunoactivated autologous leukocytes.

The culture medium can be one normally employed in the art, which are selected from a variety of preparations including alpha-MEM, DMEM-F12, and other commercially available formulations such as mesenchymal growth media from Lonza^{®}, Macropharma^{®} or Stem Cell Technologies^{®}. In one embodiment of the present invention the culture medium employed is DMEM (Dulbecco's Modified Eagle's medium) culture medium.

The fractions obtained both by enzymatic digestion and by gradient centrifugation are cultured in polystyrene culture flasks, which promotes cell adhesion, one of the main properties of MSCs of any origin. MSCs are cultured in media enriched with immunomodulation-stimulating factors, mostly FBS, human serum, platelet-rich plasma and hPL, according to the specific protocol for each laboratory.

An essential aspect in the manufacture of cell-based products is the use of immunomodulation-stimulating factors supplements that meet the criteria for use in humans. Some commercially available supplements include FBS, and other mesenchymal cell-specific growth supplements manufactured by Lonza^{®}, Macropharma^{®} or Stem Cell Technologies^{®} that have the advantage of not containing xenogenic components and are certified for use in human drug manufacturing. Other in-house preparations include the use of human serum or serum converted from platelet-poor human plasma, which has also been shown to support MSC growth, albeit with lower efficiencies when compared to hPL.

According to the present disclosure, a supplement comprising soluble factors on which isolated UC-MSCs are highly dependent to proliferate and survive is employed. These factors are fibroblast growth factor (FGF), the platelet-derived growth factor (PDGF) family AA, BB and AB, epithelial growth factor (EGF), TGFα factor, angiogenin, vascular endothelial growth factor (VEGF), and the CXCL chemokines and the chemokines CXCL2, CCL5. In turn, these factors induce a significant increase in cell vitality and cell division capacity, which in the long run confer a longer shelf life to the cell product.

In one embodiment of the present invention, the DMEM culture medium is enriched with a supplement of immunomodulation-stimulating factors including, but not limited to, those soluble molecules present in platelet-rich human plasma and its final derivative, hPL. In a particular embodiment, the DMEM culture medium is supplemented with hPL between 5 and 15% v/v of the culture medium. In a more particular embodiment, hPL is at a 10% v/v concentration in the culture medium.

### Obtaining hPL

For the purposes of the present invention, hPL is obtained from platelets of healthy blood donors (A+, B+, O+ and O-), by a sequence of freezing and thawing cycles to induce platelet lysis. Platelet lysates are centrifuged, and the supernatants are filtered, divided into aliquots and stored until use. For the culture of UC-MSCs, 5 to 10 mL of supplemented medium are added. The positivity of the cultures is evaluated 48 hours after this process. The culture medium is renewed every third day, changing half the volume of medium to fresh medium until the cells reach 75-85% confluence. At the end of this first culture phase, the culture is trypsinized and after inactivation of trypsin with supplemented medium, it is centrifuged, and the cell pellet is suspended in 10 mL of supplemented medium and seeded in a Petri dish for cell culture.

### MSC expansion

The culture with supplemented medium is carried out for 10 to 17 days, until a cell suspension is obtained, which is cultured for another 4 to 7 days, to obtain a final preparation called "master cell bank - MCB". This MCB is cryopreserved by a method known in the art. In a particular embodiment, the cryopreservation method comprises the use of a saline phosphate buffer, DMEM medium or physiological solution, a stabilizer (between 12.5 and 15% v/v) and an organic solvent acting as a cryopreservative (10% v/v). In an even more particular modality, the stabilizer is bovine serum albumin, and the organic solvent is dimethyl sulfoxide (DMSO).

A new cell culture is generated from the MCB using the same formulation of medium used previously. The culture is carried out under standard conditions comprising incubation at 37°C, 5% CO₂, and 95% humidity and normal O₂ pressure (19-21%) until the cell number indicated for the final cell application is obtained, which should be between 3 and 5×10⁶ cells per milliliter. This "drug product - DP" is cryopreserved for quality control performed on each batch. Once this process is carried out according to (production batches release) and in response to a specific clinical demand, the preparation is thawed and packaged (formulated) in its final presentation 24 to 48 hours prior to its final application.

### Induction of the immunomodulatory therapeutic effect of UC-MSCs

According to the present disclosure, during the DP preparation period, cells are stimulated to acquire an immunomodulatory phenotype from exposure to hPL-supplemented culture medium or, alternatively, to stimulation with cytokines or leukocytes from the same patient, which have been previously immunoactivated in order to induce an anti-inflammatory state prior to their final application.

UC-MSCs stimulated by the method of the present invention, acquire a previously undescribed state (phenotype) associated with a potent immunomodulatory effect characterized by direct inhibition of T-lymphocyte proliferation and induction of an immunomodulatory phenotype of human monocytes/macrophages. In an inflammatory state characteristic of different chronic inflammatory conditions (e.g., autoimmune diseases), UC-MSCs activate various immunomodulatory mechanisms through the induction of immunomodulatory factors, cell-cell contact, polarization of macrophages towards immunomodulatory states, and the activation of immune checkpoint mechanisms in activated T lymphocytes. These effects were found in immunoactivated UC-MSCs by increased expression of: soluble IL-2 receptor (slL-2r), CD274 (PD-L1), TIM-1 and CD131, in addition to other previously described markers. These cells in an anti-inflammatory state express about 6000 genes involved in numerous pathways controlling the immune response, survival, etc.

Relevantly, the anti-inflammatory and immuno-modulatory phenotype induced in MSCs by the stimulation method described here is characterized by the increased expression of programmed death factor ligand 1 (PD-L1) and the production and release of immunomodulatory factors such as interleukin (IL)-6, granulocyte and granulocyte-macrophage colony stimulating factor (G-CSF and GM-CSF), hepatocyte growth factor (HGF), which ultimately impact on the modulation of immune responses of the lymphoid and myeloid cellular compartment in the context of tissue inflammation.

In a particular embodiment of the present invention, the method of stimulation leading to the generation of the immunomodulatory phenotype of a UC-MSC product comprises the expansion of UC-MSCs in a supplemented medium prepared by an in-house methodology based on a preparation of hPL enriched with factors such as basic fibroblast growth factor (bFGF), RANTES, epidermal growth factor (EGF), macrophage-inducing factor (MIF), and tumor growth factor alpha (TGFα).

In another embodiment of the present invention the effect can be enhanced by stimulating UC-MSCs with cytokines (which, for purposes of the present invention are selected from, without limitation, IL1 alpha, IL1 beta, TNF alpha, IFN-gamma, LPS or mixtures thereof), or the addition to the final product of activated receptor leukocytes.

Both the stimulation with cytokines and the final addition of previously immuno-activated recipient leukocytes comprise a final stage of product manufacturing. In one embodiment employing cytokines, the final phase comprises the addition of each recombinant protein at a concentration between 5-50 ng/mL for 24-48 hours prior to the final product formulation. In an embodiment using activated leukocytes from the final recipient, the process involves obtaining a peripheral blood sample from the patient, isolation of MNCs and subsequent incubation for 24 hours with phytohemagglutinin or antibodies directed against the CD3 receptor of the lymphocytes to immunoactivate the lymphocyte and mononuclear component. After incubation, the immunoactivated MNC suspension is added to the final UC-MSC preparation and incubated for an additional 24 to 48 hours before final packaging.

### Pharmaceutical composition

The clinical use of MSCs has established itself as one of the most widely applied cell therapies in the clinical setting for a wide variety of acute and chronic clinical conditions. Although MSCs from different tissue sources have been described, MSCs from bone marrow and adipose tissue have been mostly applied in the clinical setting, essentially in an autologous use approach, i.e., the cell product is derived from the same patient to be treated, with the subsequent operational and logistical complexity, together with the delay involved in obtaining the tissue, its processing and subsequent application in a customized manner.

The development and potential application of UC-MSC-based products has opened a new window of opportunity for the generation of therapeutic cell products for allogeneic use (the donor is different from the recipient). Moreover, the possibility of generating master banks of this cell product offers an additional advantage by allowing the generation of unlimited doses available for immediate clinical use. This technology describes a chain production flow of advanced therapy products based on UC-MSCs with high reproducibility and homogeneity, with quasi-generic characteristics that will allow reproducible clinical results during their clinical application.

A pharmaceutical composition within the scope of the present invention comprises an immunomodulatory umbilical cord mesenchymal stromal cell (UC-MSC) population obtained by the method described above, immunomodulatory factors and a pharmaceutically acceptable vehicle.

The immunomodulatory MSC population according to the present invention exerts a potent anti-inflammatory effect, as a consequence of the expression of the factors mentioned above, as well as multiple repressors of the immune response identified at the transtryptomic level.

The results demonstrate that MSCs in the presence of activated human T lymphocytes induce 100% inhibition of proliferation of previously immunostimulated lymphocytes. This inhibition is associated with induction of CD25 and CD274 expression on MSCs as well as accumulation of GM-CSF, G-CSF, IL-10- IL- 6, MIG, MCP-1, IP-10 factors, the reduction of factors such as MIP-1b, IL1-Ra, TNF-alpha among others, which are related to the reduction of inflammatory macrophages (type I macrophages), the increase of immunomodulatory macrophages (type II macrophages), and the induction of terminal phenotype T lymphocytes (TEMRA).

The pharmaceutical composition of the present invention can be formulated in different formats, depending on the final application. In one embodiment, the composition is formulated as an injection in volumes of 5 to 10 milliliters for local infiltration. In another particular embodiment, the composition is formulated as a cell suspension (intravenous infusion bag of up to 100 mL) with a cell quantity corresponding to 2-5 million cells per kg of patient weight, for systemic application.

A pharmaceutically acceptable vehicle in accordance with the present invention is selected, but is not limited to a balanced crystalloid solution similar to human plasma in its electrolyte content, osmolality and pH between 7.2 and 7.4; plus the addition of human albumin; human AB serum or some other source of macromolecules that provide support and stability to the living cell component; and buffer solutions such as phosphates, isotonicity agents such as glucose, sodium chloride and mixtures thereof. In a particular embodiment, the pharmaceutically acceptable vehicle is a PlasmaLyte-type crystalloid solution, with an addition of 2% human albumin.

### Method of treatment

In accordance with the present invention, the pharmaceutical composition comprising a population of immunomodulatory MSCs, immunomodulatory factors and a pharmaceutically acceptable vehicle may be administered in a therapeutically effective amount to a patient in need thereof for the treatment of a condition of an autoimmune nature or associated with acute or chronic inflammation.

The high potency anti-inflammatory cell-enriched product can be injected locally or infused intravenously in patients with various chronic inflammatory conditions of an autoimmune nature selected from, but not limited to, arthrosis, osteoarthrosis, osteoarthrosis of the knee, osteoarthrosis of the hip, rheumatoid arthritis, systemic lupus erythematosus, multiple sclerosis, scleroderma, among others.

The product can be administered by various parenteral routes, such as local intra-articular injection or systemic intravenous route. In one embodiment, for intra-articular application the dose varies between 5 and 20×10⁶ cells in total. In another embodiment for intravenous application, the dose varies between 2 and 10×10⁶ cells per kg of patient weight. The application regimen can be a single dose or monthly applications up to a maximum of 3-4 applications according to the clinical condition specified.

In a particular embodiment, the method of treatment using MSCs stimulated with immunoactivated autologous leukocytes comprises obtaining the leukocytes by venipuncture from 15-20 mL of peripheral blood, 48-72 hours prior to the final application of the cell therapy product. During this period, the isolation of MNCs, their culture for 24 h with the immunoactivator, and the final addition of this cell suspension to the final UC-MSC product are carried out. The final administration is carried out in one of the presentations described above at a dose dependent on the final number of UC-MSCs.

The present invention will be described in detail through the following examples, which are provided for illustrative purposes only and are not intended to limit its scope.

### EXAMPLES

### Example 1. Obtaining a population of immunomodulatory UC-MSCs

### UC-MSC insulation

This study was approved by the Research Ethics Committee of the District Secretary of Health of Bogotá, Colombia and written consent was obtained from the umbilical cord (UC) donors. The UC was collected under aseptic conditions from women after a full-term pregnancy (cesarean section or normal vaginal delivery).

Three methods for UC processing were compared: tissue enzymatic digestion, WJ enzymatic digestion and WJ explant. For WJ explant processing, the UC was cut into 3 cm pieces and the residual blood was washed three times with sterile phosphate buffered saline (PBS) 1X (Gibco, Life Technologies, Carlsbad, CA, USA) containing 1% penicillin/streptomycin (P/S) 10,000 U/mL (Gibco, Life Technologies, Carlsbad, CA, USA). Each piece of cord was cut longitudinally, the cord vessels were removed, and the epithelial layer was discarded. The WJ was removed, cut and placed directly into 35 mm tissue culture plates containing low glucose DMEM medium (Gibco, Life Technologies, Carlsbad, CA, USA), supplemented with 10% hPL. These cultures were maintained at 37 °C in a humidified atmosphere with 5% CO₂. Upon reaching 75% to 85% confluence, cells were released from the plate using 0.25% trypsin (Gibco, Life Technologies, Carlsbad, CA, USA) for 3 minutes, they were counted and returned to the plates.

For enzymatic digestion of UC, 3 cm long sections of UC were cut into small pieces, added to a solution containing 0.075% collagenase I (SigmaAldrich, St Louis, MO, USA) and incubated for 60 minutes at 37 °C under constant stirring. The cell suspension was passed through a 100 µm mesh cell filter, resuspended in DMEM supplemented with hPL and seeded in a 6-well plate. Non-adherent cells were removed after 6 hours of culture. For enzymatic digestion of WJ, the WJ was removed from the UC and digested in 0.075% collagenase I solution (SigmaAldrich, St Louis, MO, USA) for 60 minutes at 37 °C under constant stirring. Cells were passed through a 100 µm mesh cell filter and resuspended in DMEM supplemented with 10% hPL and 1% P/S. Cells were seeded in 6-well plates and maintained until confluence. Using these three methodologies, a 100% yield was obtained when using enzymatic digestion or WJ explant (FIG. 1A). Conversely, tissue digestion showed a yield of less than 80%. Once cultures of both WJ and whole UC tissue were initiated, migration and growth (cell derivation) were observed between 5 and 17 days **(****FIG. 1B****).** WJ explant resulted in shorter derivatization times with respect to WJ or whole tissue digestion.

### Human platelet lysate (hPL)

In the development of the present invention, the production of hPL derived from platelet bags was standardized for cell culture. hPL was obtained in the institutional blood bank from platelets of healthy blood donors (A+, B+, O+ and O-). 45 mL aliquots (3 donors) were frozen at -80 °C and subjected to two freeze-thaw cycles to induce platelet lysis. Platelet lysates were then centrifuged at 4,000 g for 10 min and the supernatants were filtered through 0.2 µm membranes, divided into aliquots and stored at -80 °C until use. The hPL from three donors was combined and added to DMEM at a 10% concentration for use in MSC culture. Recombinant human heparin was added at a final concentration of 16 IU/mL.

### UC-MSC culture and expansion in culture medium supplemented with immunomodulation-stimulating factors.

To test the ability of hPL batches to support UC-MSC growth, cells from six donors were maintained in culture for 7 passages in the presence of DMEM supplemented with 10% hPL. Media supplemented with 10% FBS were used as control. Population doubling levels (PDL) for each passage were calculated using the formula X = [Iog10 (NH) -log10 (Nl)] / log10 (2), where NI is the number of inoculum and NH is the number of cells harvested. The increase in population doubling was also calculated by aggregating the PDL level for each passage to obtain the cumulative population doubling (CPD). No significant differences were found in PDL and CPD values in cultured MSCs exposed to hPL obtained from the A+, B+, O- and O+ groups **(****FIG. 1C and 1D****).** In addition, stable cell growth kinetics were found in all passages evaluated, with an average PDL of 3.2. Conversely, cells cultured in FBS-supplemented media exhibited significantly lower PDL values. Finally, higher, and more stable cell proliferation rates of MSCs exposed to hPL-supplemented culture medium resulted in higher CPD values compared to MSCs cultured in FBS media (p<0.05).

The presence of various immunomodulation-stimulating factors contained in various hPL batches was evaluated by comparing immunomodulation-stimulating factors, inflammatory cytokines, chemokines and growth factors and their uptake by UC-MSCs. Overall, the hPL batches evaluated showed a homogeneous effect on the stimulation of MSC-WJ proliferation and a homogeneous content of growth factors and cytokines, when compared with FBS **(****FIG. 2A****).** The various hPL batches contained higher levels of FGFb, CCL5, EGF, MIF and TGF relative to the standard supplement and were actively consumed by UC-MSCs over a culture period of 5 days **(****FIG. 2B****).** Relevantly, the presence of hPL but not standard supplementation induced significant expression of HGF, GM-CSG, IGFBP-1 and IGFBP-2, factors that have been recognized as potent immunomodulators **(****FIG. 2C****).**

UC-MSCs were harvested and resuspended at a final concentration of 1×10⁶ cells/ml. Cells were transferred to 1000 µl of pre-cooled medium containing DMEM and 10% dimethyl sulfoxide (DMSO) supplemented with 30% hPL (n = 22) or 30% FBS (n = 22). For each group, cells were cryopreserved using a CryoMed controlled-rate freezer at a freezing rate of 1 °C per minute or by placing cryovials in pre-cooled isopropanol racks (Mr. Frosty, Nalgene^{®}) and transferring them to the freezer at -80 °C 24 h before final storage in liquid nitrogen (LN₂). Cell samples were stored for one month until thawing. To assess viability and recovery, UC-MSCs were thawed and washed. Viable and dead cells were counted with a Neubauer chamber after staining with trypan blue (0.4%, Life Technologies). Cells were further seeded in 25 cm² tissue culture flasks (Corning, USA) at a density of 4.6×10³ cells/cm² and seeded to confluence to obtain PDL, CPD and PDT values after thawing. Additionally, UC-MSCs were characterized for their differentiation potential toward osteogenic, adipogenic and chondrogenic lineages **(****FIG. 5A****).** Expression of cell surface antigens related to MSCs was assessed by flow cytometry using membrane markers, CD90 (APC), CD73 (PECy7), CD105 (PE), CD45 (APC / Cy7), CD34 (PerCP-Cy5.5), HLA-DR (Pacific Blue) and HLA-ABC (FITC). Cells were incubated for 30 min at 4 °C, centrifuged at 300 g for 6 min and resuspended in 0.2 mL PBS. The procedure was performed on a FACSCanto II flow cytometer (Becton Dickinson, San Jose, USA) and the data were analyzed with the FlowJo vX.7.0 data analysis software package (TreeStar, USA). Likewise, the phenotype of UC-MSCs isolated from different donors was evaluated based on the identification of the mesenchymal identity phenotype evaluated by the positivity of the markers CD73, CD90, CD105 and negativity of the hematopoietic markers CD45, CD34, HLA-DR and CD31 **(****FIG. 5B****).** Finally, it was possible to demonstrate an increase in the expression of the immunomodulation marker CD274 (70-90%) in expanded UC-MSCs in the presence of hLP.

### Immunomodulatory effect of UC-MSCs

Direct (cell-to-cell contact) and indirect (transwell) co-cultures were evaluated. PBMCs were thawed and cultured for 24 hours. Subsequently, PBMCs were stimulated with 1 µg/mL of anti-CD2, -CD3, -CD28 (αCD3/CD28) phytohemagglutinin (PHA) T-cell activation beads (Miltenyi Biotec GmbH, Bergisch Gladbach, Germany). UC-MSCs of up to 6 passages were adjusted to 5×10⁴ cells/well in a 24-well plate and cultured for 5 hours. After this time, the UC-MSC medium was removed and 5×10⁵ PBMC cells were added in RPMI-1640 supplemented with 10% FBS. Plates were incubated for 120 hours at 37 °C with 5% CO₂. For indirect co-cultures, UC-MSCs were seeded at the bottom of the well and stimulated PBMCs were seeded on top of transwell inserts (pore size of 1 µm). Each assay was repeated three times and supernatants were collected and stored at -80 °C for subsequent cytokine assays. The inhibitory effect of UC-MSCs on lymphocyte proliferation was measured by positive CD3 counts using CountBright absolute counting beads (Invitrogen, Life Technologies, Carlsbad, CA, USA). The number of cells per microliter was calculated as a function of the number of cells counted x bead concentration / number of beads counted. After incubation, suppression of approximately 90% of PHA-activated CD3+ lymphocytes was observed in dependent and independent contact with UC-MSCs (FIG. 3). The presence of UC-MSCs induced significant suppression of anti-CD3/CD28-activated MNCs in both cell-dependent and cell-independent contact.

### Example 2. Characterization of immunomodulatory factors expressed by MSCs

The concentration of cytokines, chemokines, and immunomodulation-stimulating factors in cell supernatants of UC-MSCs in the immunomodulated state was assessed by flow cytometry bead assay technology. Cytokines (GM-CSF, IL-1α, IL-1β, IL-6, IL-8, TNF-α, IFN-γ, LPS, IL-2, IL-2R, IL-7, IL-12, IL-15 and IL-17), anti-inflammatory cytokines (IL-1RA, IL-4, IL-5, IL-10, IL-13 and IFN-α), chemokines (eotaxin, IP-10, MCP-1, MIG, MIP-1α, MIP-1β and RANTES), angiogenic immunomodulation-stimulating factors (VEGF) and immunomodulatory and immunomodulation-stimulating factors (EGF, HGF, bFGF, G-CSF) were determined by the Invitrogen^{™} Cytokine 30-Plex Human Panel assay (Carlsbad, California, USA). The procedure was performed according to the manufacturer's instructions and the concentration was reported in pg/mL. Among the main factors released by UC-MSCs during immunomodulation, a significant increase in G-CSF, MCP-1, IL-6, IL-10 and IP-10 was detected **(****FIG. 4****).** This expression profile of immunomodulatory factors was similar when MNCs were stimulated with PHA or aCD3/CD28. Likewise, this identified secretory profile was present in UC-MSCs that were in direct contact with MNCs or in independent contact (transwell). Additionally, from transcriptome analysis in UC-MSCs in immunomodulated state (exposed to PHA-activated MNCs), the differential expression of 6,077 genes was identified **(****FIG. 3C and 3D****).** Of these, a group of selected genes corresponding to signals inducing immunomodulation processes were found to be significantly activated in UC-MSCs during immunomodulation (C2, FIG. 3E).

### Example 3. Preconditioning of UC-MSCs with cytokines induces immunomodulatory signals

The cytokine and immunomodulation-stimulating factor content of different hPL preparations was determined by quantitative cytokine arrays using the Luminex Human Cytokine 30-plex commercial assay (Invitrogen, Carlsbad, California, USA), in accordance with the manufacturer's instructions. For identity and functional phenotyping of UC-MSCs, cells were cultured and stimulated in different conditions such as PHA, αCD3/CD28 or cytokine cocktail consisting of IL-1b (50 ng/mL) and TNF-a (50 ng/mL) or activated PBMNC, and the expression of PDL-1, PDL-2, CD80 and CD86 was evaluated. Isotype control was included for all flow cytometry analyses. The FACSCanto II instrument (Becton Dickinson, San Jose, USA) was used, and the data set was analyzed using the FlowJo vX.7.0 data analysis software package (TreeStar, USA). UC-MSCs from different donors exposed to inflammatory cytokines induced CD25 (soluble IL2R) expression, as well as PD-L1 and PD-L2 expression 48 hours post-incubation **(****FIG. 4****),** indicating the induction of a highly immunomodulatory phenotype through cell preconditioning by incubation with cytokines.

### Example 4. UC-MSCs exposure to immunoactivated leukocytes

It was previously demonstrated that the UC-MSC population expanded by the method developed in this invention is highly enriched with cells with a potent immunomodulatory phenotype that themselves exert a suppressive effect on the proliferation of activated lymphocytes. Additionally, evidence accumulated during the development of the method according to the present invention suggests that the immunoactivation of leukocytes and subsequent exposure to UC-MSCs induces a significantly increased suppressive response by these stromal cells.

Accordingly, preconditioning of UC-MSCs can be performed by exposing UC-MSCs to immunoactivated leukocytes prior to the final formulation, release and clinical application of the drug product.

According to the present disclosure, the immunoactivated leukocytes can be obtained from a patient's peripheral blood sample (minimum 10 mL), 48-72 hours prior to cell infusion. The sample is then transferred to the cell production laboratory. A bout 0.7-1 × 10⁷ PBMCs will be obtained from this blood sample, which are immediately activated with αCD3/CD28 antibodies to induce lymphoproliferation and induction of inflammatory cytokines. PBMCs are incubated for 24 hours with phytohemagglutinin, or antibodies directed against the CD3 receptor of lymphocytes to immunoactivate the lymphocyte and mononuclear component, and then they are transferred to the UC-MSC cell suspension from the cell master bank, followed by further incubation for 24 hours until their final formulation.

Finally, this activated UC-MSC/PBMC cell suspension-based product will be released and transferred to the clinical service.

### Example 5. Composition of an immunomodulatory UC-MSC-based advanced therapy drug product

The cell product developed herein arises from an expansion process duly controlled and monitored to ensure product homogeneity. The production process includes the generation of a master cell bank and the subsequent expansion of this bank into individual doses of immunomodulatory MSCs of 20-40 million cells per unit, cryopreserved at -190 °C.

The customized drug product based on a preconditioning strategy with inflammatory cytokines consists of an aliquot of the UC-MSC master cell bank, the cell suspension of which is transferred into a culture flask and immediately exposed in culture to a mixture of recombinant proteins corresponding to selected inflammatory factors, including IL-1α and/or IL-1β in combination with TNFα at concentrations between 10-50 ng/mL final volume or immunoactivated leukocytes from a peripheral blood sample from the patient. The UC-MSC product treated with inflammatory factors is incubated for up to 24 hours.. For the final formulation and release process for clinical use, the product is resuspended, dosed in a format that can be a 10 mL syringe for local application or a 100-200 mL volume bag for systemic application that is sent to the clinical unit. This cell suspension, duly characterized in terms of purity, viability, potency and identity is contained in a crystalloid-type solution containing different electrolytes, including, but not limited to, sodium (140 mmol/l), potassium (5 mmol/l), magnesium (1.5 mmol/l), chloride (98 mmol/l), acetate (27 mmol/l) and gluconate ions (23 mmol/l), and an addition of 2-5% human albumin.

It is important to mention that the strategy of stimulating UC-MSCs with immunoactivated leukocytes as mentioned in Example 4 does not generate a risk of additional allogeneic reactions derived from the transfer of leukocytes (especially lymphocyte populations) into the recipient, because the PBMC population is autologous. In addition, as observed experimentally, these leukocytes are in a state of total inhibition resulting from the immunosuppressive effect of the mesenchymal cells included in the drug product.

### Example 6. Application of the immunomodulatory UC-MSC composition, therapeutic uses, and methods

The clinical application of MSC-based advanced therapy drug products is increasing in different clinical scenarios with varying degrees of therapeutic efficacy according to the targeted pathology and the selected cell source. Mainly, the UC-MSCs have recently been introduced for the therapeutic management of graft-versus-host disease (GVHD), in particular after allogeneic hematopoietic progenitor cell transplantation. Additionally, there is accumulated clinical experience in some autoimmune conditions such as rheumatoid arthritis, systemic lupus erythematosus, and multiple sclerosis. Under these conditions, the UC-MSC cell product is administered intravenously by venous puncture and therefore its application is considered as "systemic" use. Regarding the dose of administration, although there are limited accumulated clinical applications, a dose of 2-5×10⁶ cells per kilogram of weight of the treated patient, per dose, is generally considered. In the case of GVHD, the cell suspension has been administered at a frequency of one dose per month, with a total dosage of 4-6 doses; however, the treatment regimen should be tailored to the condition and expected outcome. As for the setting in autoimmune disease, the treatment regimen is similar in terms of dose, frequency and total dose. In general terms, the use of MSCs has demonstrated high safety in terms of adequate tolerance to administration and absence of severe or serious adverse effects.

Another promising clinical application of MSCs has to do with their use in the treatment of arthrosis and osteoarthrosis, mainly of the knee and hip. In these clinical scenarios, the greatest efficacy observed is associated with its local application, directly at the lesion site (mainly intra-articular injection). For this type of application, the cell suspension is formulated in final presentation as an injection, for immediate application (no more than 12 hours post-release). In this case, the application regimen consists of intra-articular injections of 10-20×10⁶ cells per injection. 1 to 4 injections per year have been reported, spaced between 3 and 12 months per application. Again, local use of MSC-based cell therapy products have not resulted in severe/serious adverse effects with this type of application and their clinical efficacy is positive.

## Claims

1. A method for obtaining an immunomodulatory mesenchymal stromal cell (MSC) population, comprising the steps of:
a) growing and expanding MSCs in culture medium supplemented with immunomodulation-stimulating factors; and optionally,
b) adding cytokines or exposing to immunoactivated autologous leukocytes.

2. The method of claim 1, wherein the immunomodulation-stimulating factor supplement comprises human platelet lysate between 5 and 10% v/v of the culture medium.

3. The method of claim 1, wherein in step b) the cytokines are selected from the group consisting of IL1-alpha, IL1-beta, TNF-alpha, IFN-gamma, LPS or mixtures thereof.

4. The method of claim 1, wherein step b) has a duration of between 24 and 120 hours.

5. The method of claim 1, wherein in step b), the MSCs express immunomodulatory factors selected from the group consisting of HGF, CD25, CD273, CD274, TIM-1, CD131, G-CSF, GM-CSF, IL-10- IL-6, MIG, MCP-1 and IP-10.

6. A pharmaceutical composition comprising:
an immunomodulatory mesenchymal stromal cell (MSC) population obtained by the method of claim 1;
immunomodulatory factors; and
a pharmaceutically acceptable vehicle.

7. The composition of claim 6, wherein the immunomodulatory factors are selected from the group consisting of: soluble receptor of HGF, CD25, CD273, CD274, TIM-1, CD131, G-CSF, GM-CSF, IL-10- IL-6, MIG, MCP-1, IP-10, and mixtures thereof.

8. The composition of claim 7, wherein the immunomodulatory factors are expressed by MSCs.

9. A method of treating a condition of an autoimmune nature or associated with acute or chronic inflammation, wherein the method comprises administering a therapeutically effective amount of the pharmaceutical composition of claim 6 to a patient in need thereof.

10. The method of claim 9, wherein the autoimmune condition or condition associated with acute or chronic inflammation is selected from the group consisting of osteoarthrosis of the knee, rheumatoid arthritis, systemic lupus erythematosus, multiple sclerosis, scleroderma, graft-versus-host disease, among others.
